# FASCICULE DE BREVET EUROPEEN

(11) **EP 2 431 360 B1**
(45) Date de publication et mention de la délivrance du brevet: **17.04.2013**
(21) Numéro de dépôt: 11290413.1
(22) Date de dépôt: 15.09.2011
(51) Int. Cl.: C07D 291/08, A61K 31/551, A61P 25/00

(54) **Derives de dihydrobenzoxathiazepines, leur procede de preparation et les compositions pharmaceutiques qui les contiennent ainsi que leur utilisation comme modulateurs des recepteurs AMPA**
Dihydrobenzoxathiazepinderivate, ihr Herstellungsverfahren und pharmazeutische Zusammensetzungen, die sie enthalten unde deren Verwendung als AMPA-Modulatoren
Derivatives of dihydrobenzoxathiazepine, method of preparing same and pharmaceutical compositions containing them as well as their uses as modulators of AMPA receptors

(30) Priorité: 16.09.2010 FR 1003683
(43) Date de publication de la demande: 21.03.2012
(73) Titulaire: Les Laboratoires Servier, 92284 Suresnes Cedex (FR)
(72) Inventeur: Cordi, Alexis, 92150 Suresnes (FR); Desos, Patrice, 92270 Bois-Colombes (FR); Lestage, Pierre, 78170 La Celle Saint Cloud (FR); Danober, Laurence, 78360 Montesson (FR)

(56) Documents cités:
- WO-A1-02/100865
- WO-A1-2006/125972
- WO-A2-99/42456

## Description

La présente invention concerne de nouveaux dérivés dihydrobenzoxathiazépines, leur procédé de préparation, les compositions pharmaceutiques qui les contiennent ainsi que leur utilisation par la fabrication de médicaments utiles en tant que modulateurs allostériques positifs des récepteurs AMPA.

Il est désormais reconnu que les aminoacides excitateurs et tout particulièrement le glutamate, jouent un rôle crucial dans les processus physiologiques de plasticité neuronale et dans les mécanismes sous-tendant l'apprentissage et la mémoire. Des études pathophysiologiques ont indiqué clairement qu'un déficit de la neurotransmission glutamatergique est associé étroitement avec le développement de la maladie d'Alzheimer (Neuroscience and Biobehavioral Reviews, 1992, 16, 13-24; Progress in Neurobiology, 1992, 39, 517-545).

Par ailleurs, certains travaux ont démontré durant les dernières années l'existence de sous-types réceptoriels aux acides aminés excitateurs et de leurs interactions fonctionnelles (Molecular Neuropharmacology, 1992, 2, 15-31).

Parmi ces récepteurs, le récepteur à l'AMPA ("α-amino-3-hydroxy-5-methyl-4-isoxazole-propionic acid") apparaît être le plus impliqué dans les phénomènes d'excitabilité neuronale physiologique et notamment dans ceux impliqués dans les processus de mémorisation. Pour exemple, l'apprentissage a été montré comme étant associé à l'augmentation de la liaison de l'AMPA à son récepteur dans l'hippocampe, l'une des zones cérébrales essentielles aux processus mnémocognitifs. De même, les agents nootropes tels que l'aniracetam ont été décrits comme modulant positivement les récepteurs AMPA des cellules neuronales (J. Neurochemistry, 1992, 58, 1199-1204).

Dans la littérature, des composés de structure benzamide ont été décrits pour posséder ce même mécanisme d'action et pour améliorer les performances mnésiques (Synapse, 1993, 15, 326-329). Le composé BA 74, en particulier, est le plus actif parmi ces nouveaux agents pharmacologiques.

Plus récemment, la demande de brevet WO 99/42456 décrit des dérivés benzothiadiazine et benzothiadiazépine en tant que modulateurs des récepteurs AMPA et la demande de brevet WO 02/100865 décrit des dérivés benzoxazépines possédant une activité facilitatrice sur le courant AMPA.

Les dérivés dihydrobenzoxathiazépines, objets de la présente invention, sont nouveaux et constituent de puissants modulateurs allostériques positifs des récepteurs AMPA.

Plus spécifiquement, la présente invention concerne les composés de formule (I) : 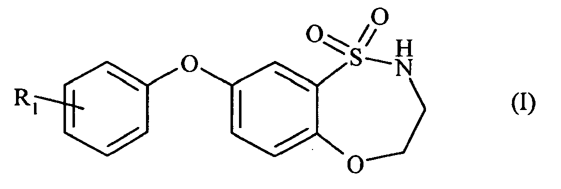 dans laquelle R₁ représente un atome d'hydrogène, un groupement cyano, un groupement alkoxycarbonyle (C₁-C₆) linéaire ou ramifié, un groupement alkyl(C₁-C₆)sulfonylaminoalkyle (C₁-C₆) linéaire ou ramifié, un groupement N-hydroxycarboximidamide, ou un groupement hétérocyclique,
leurs énantiomères et leurs diastéréoisomères, ainsi que leurs sels d'addition avec un acide ou une base pharmaceutiquement acceptable,
étant entendu que le terme groupement hétérocyclique désigne un groupement aromatique monocyclique à 5 chaînons, contenant un à quatre hétéroatomes, identiques ou différents, choisis parmi azote, oxygène et soufre, ledit groupement hétérocyclique pouvant être éventuellement substitué par un ou plusieurs substituants, identiques ou différents, choisis parmi alkyle (C₁-C₆) linéaire ou ramifié ou polyhalogénoalkyle (C₁-C₆) linéaire ou ramifié.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléique, citrique, ascorbique, oxalique, méthanesulfonique, benzènesulfonique, camphorique. Parmi les bases pharmaceutiquement acceptables, on peut citer à titre non limitatif l'hydroxyde de sodium, l'hydroxyde de potassium, la triéthylamine, la tertbutylamine.

R₁ représente de manière préférée un groupement hétérocyclique.

Plus préférentiellement, R₁ représente un groupement hétérocyclique monocyclique aromatique à 5 chaînons contenant au moins un atome d'azote, ledit groupement pouvant être éventuellement substitué par un ou plusieurs substituants, identiques ou différents, choisis parmi alkyle (C₁-C₆) linéaire ou ramifié ou polyhalogénoalkyle (C₁-C₆) linéaire ou ramifié.

R₁ représente avantageusement les groupements pyrrolyl, pyrazolyl, imidazolyl, triazolyl, tetrazolyl, isoxazolyl, oxazolyl, isothiazolyl, thiazolyl, thiadiazolyl, dithiazolyl, et oxadiazolyl, chacun des groupements pouvant être éventuellement substitués par un ou plusieurs substituants, identiques ou différents, choisis parmi alkyle (C₁-C₆) linéaire ou ramifié ou polyhalogénoalkyle (C₁-C₆) linéaire ou ramifié.

Sont plus particulièrement préférés les composés pour lesquels R₁ représente les groupements oxadiazolyl et thiazolyl et, plus particulièrement, les groupements 1,2,4-oxadiazolyl et 1,3-thiazolyl, chacun des groupements préférés pouvant être éventuellement substitués par un ou plusieurs substituants, identiques ou différents, choisis parmi alkyle (C₁-C₆) linéaire ou ramifié ou polyhalogénoalkyle (C₁-C₆) linéaire ou ramifié.

Les substituants préférés du groupement hétérocyclique sont le groupement méthyle ou le groupement trifluorométhyle.

Le groupement R₁ est préférentiellement en position méta ou para du noyau phénoxy qui le porte. Avantageusement, le groupement R₁ est en position méta.

Les composés préférés selon l'invention sont :
- le 8-[3-(5-méthyl-1,2,4-oxadiazol-3-yl)phénoxy]-3,4-dihydro-2*H*-5,1,2-benzoxathiazépine 1,1-dioxyde ;
- le 8-[3-(3-méthyl-1,2,4-oxadiazol-5-yl)phénoxy]-3,4-dihydro-2*H*-5,1,2-benzoxathiazépine 1,1-dioxyde.

Les sels d'addition à un acide ou une base pharmaceutiquement acceptable des composés préférés de l'invention font partie intégrante de l'invention.

L'invention s'étend également au procédé de préparation des composés de formule (I) à partir du composé de formule (II) : 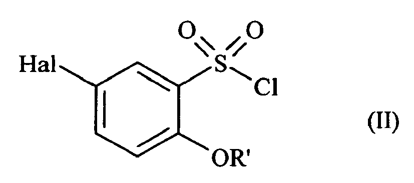 dans laquelle Hal représente un atome d'halogène, tel que le fluor, le chlore ou le brome, et R' représente un groupement alkyle (C₁-C₆) linéaire ou ramifié,
que l'on met en réaction avec de la 2-chloroéthylamine en milieu basique pour conduire au composé de formule (III) : 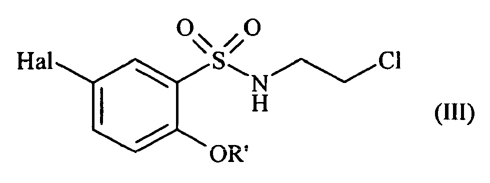 dans laquelle Hal et R' sont tels que définis précédemment,
qui subit alors l'action d'un dérivé borique pour conduire au composé de formule (IV) : 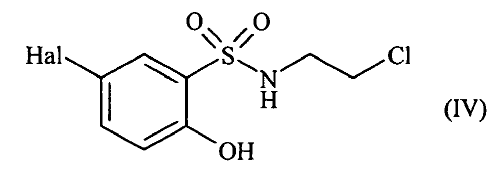 dans laquelle Hal est tel que défini précédemment,
qui est ensuite cyclisé pour conduire au composé de formule (V) : 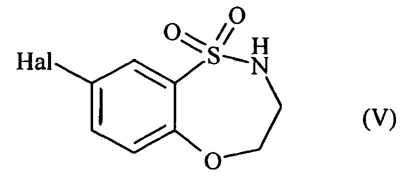 dans laquelle Hal est tel que défini précédemment,
qui subit une réaction de protection sur l'atome d'azote pour conduire au composé de formule (VI) : 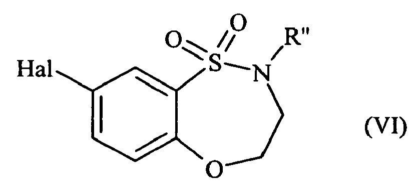 dans laquelle Hal est tel que défini précédemment et R" représente un groupement protecteur de la fonction amine, comme par exemple le groupement *tert-*butyloxycarbonyle,
que l'on transforme en acide boronique pour conduire aux composés de formule (VII) : 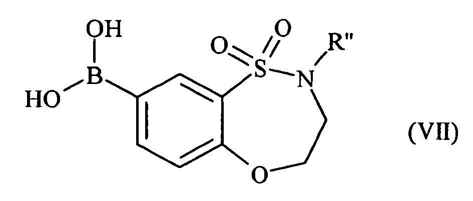 dans laquelle R" est tel que défini précédemment,
que l'on fait réagir avec un alcool de formule (VIII) : 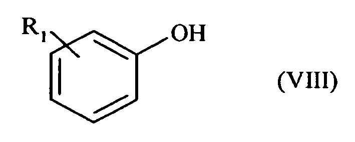 dans laquelle R₁ a la même signification que dans la formule (I),
pour conduire au composé de formule (IX), 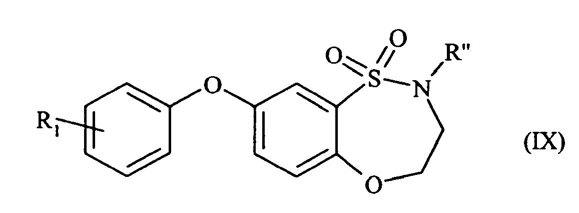 dans laquelle R₁ et R" sont tels que définis précédemment ;
qui est ensuite soumis à une réaction de déprotection de la fonction amine pour conduire au composé de formule (I),
une variante dans la préparation des composés de formule (I) consistant en l'hydrolyse du composé de formule (VII) pour conduire au composé de formule (X) : 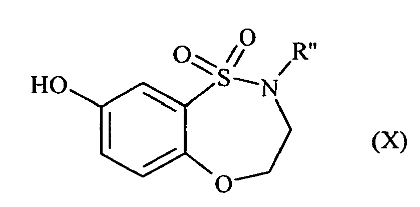 dans laquelle R" est tel que défini précédemment,
que l'on fait réagir avec un dérivé d'acide boronique de formule (XI) : 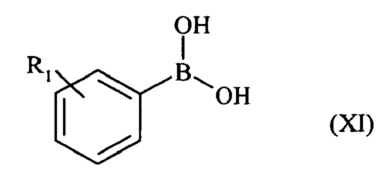 dans laquelle R₁ a la même signification que dans la formule (I),
pour conduire au composé de formule (IX),
qui est ensuite soumis à une réaction de déprotection de la fonction amine pour conduire au composé de formule (I),
une autre alternative dans la préparation des composés de formule (I) consistant, après la préparation des composés de formule (IX), en l'utilisation des réactions classiques de chimie afin de modifier, dans un deuxième temps, le substituant du noyau phénoxy,
composé de formule (I) qui peut être ensuite purifié selon une technique classique de séparation, que l'on transforme, si on le souhaite en ses sels d'addition à un acide ou à une base pharmaceutiquement acceptable et dont on sépare éventuellement les isomères, s'ils existent, selon une technique classique de séparation.

Les composés de formule (II), (VIII) et (XI) sont commerciaux ou aisément accessibles à l'homme du métier par des réactions de chimie classiques ou décrites dans la littérature. Les composés de formule (V) sont nouveaux et font également partie de l'invention à titre d'intermédiaires de synthèse des composés de formule (I).

Les composés de formule (I) selon l'invention présentent des propriétés activatrices des récepteurs AMPA qui les rendent utiles dans le traitement ou la prévention des désordres mnémocognitifs associés à l'âge, aux syndromes anxieux ou dépressifs, aux maladies neurodégénératives progressives, à la maladie d'Alzheimer, à la maladie de Parkinson, à la maladie de Pick, à la chorée d'Huntington, à la maladie de Korsakoff, à la schizophrénie, aux séquelles des maladies neurodégénératives aiguës, aux démences frontales et sous-corticales, aux séquelles de l'ischémie et aux séquelles de l'épilepsie.

L'invention s'étend aussi aux compositions pharmaceutiques renfermant comme principe actif au moins un composé de formule (I) avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les comprimés sublinguaux, les gélules, les tablettes, les suppositoires, les crèmes, les pommades, les gels dermiques, les préparations injectables, et les suspensions buvables.

La posologie utile est adaptable selon la nature et la sévérité de l'affection, la voie d'administration ainsi que l'âge et le poids du patient. Cette posologie varie de 0,01 à 1000 mg par jour en une ou plusieurs prises.

Les exemples suivants illustrent l'invention mais ne la limitent en aucune façon.

Les produits de départ utilisés sont des produits connus ou préparés selon des modes préparatoires connus.

Les structures des composés décrits dans les exemples ont été déterminées selon les techniques spectrophotométriques usuelles (infrarouge, RMN, spectrométrie de masse). Les préparations décrites ci-dessous conduisent à des produits de départ utilisés lors de la synthèse des composés de l'invention.

### Préparation 1 : 3-(5-méthyl-1,2,4-oxadiazol-3-yl)phénol

### Stade A : N',3-dihydroxybenzenecarboximidamide

A une suspension de 15,84 g (0,228 mol) de chlorhydrate d'hydroxylamine dans 75 ml de DMSO, sont ajoutés 32 ml (0,228 mol) de triéthylamine. La solution est agitée fortement pendant 30 minutes puis est diluée avec un peu de THF. Après 10 minutes d'agitation, le chlorhydrate de triéthylamine est filtré et le résidu est rincé avec du THF. Une fois le filtrat concentré (évaporation du THF), 10 g (83,9 mmol) de 3-hydroxybenzonitrile sont ajoutés à la solution obtenue. La solution est agitée à température ambiante pendant 16 heures. Après dilution à l'eau froide, le mélange est extrait 3 fois à l'acétate d'éthyle et la phase organique est lavée à la saumure. Le produit attendu est obtenu après séchage sur MgSO₄ et évaporation.

### Stade B : Acétate de 3-[N'-(acétyloxy)carbamimidoyl]phényle

A une suspension de 10,2 g (67 mmol) du produit obtenu au Stade A précédent dans 150 ml de CH₂Cl₂, sont ajoutés 28 ml (0,201 mol) de triéthylamine. 13,9 ml (0,147 mol) d'anhydride acétique sont additionnés goutte à goutte et la solution est agitée une nuit à température ambiante. Après 3 lavages à l'eau puis à la saumure, la phase organique est séchée sur MgSO₄ et évaporée. Le solide obtenu est repris dans de l'éther isopropylique puis filtré et séché pour conduire au produit du titre.

### Point de fusion : 128 °C

### Microanalyse élémentaire :

| | *C* | *H* | *N* |
|---|---|---|---|
| *% théorique* | *55, 93* | *5, 12* | *11, 86* |
| *% expérimental* | *55,94* | *5,13* | *11,71* |

### Stade C : Acétate de 3-(5-méthyl-1,2,4-oxadiazol-3-yl)phényle

A une solution de 9,40 g (39,8 mmol) du produit obtenu au Stade B précédent dans 300 ml de toluène, sont ajoutés 150 mg d'acide para-toluène sulfonique et le milieu réactionnel est chauffé au reflux avec un système dean-stark pendant 12 heures. Le produit du titre est obtenu après évaporation au rotavapor du toluène.

### Point de fusion : 94°C

### Stade D: 3-(5-méthyl-1,2,4-oxadiazol-3-yl)phénol

Une solution de 8,55 g (39,18 mmol) du produit obtenu au Stade C précédent dans 120 ml d'une solution de HCl 1 N est chauffée 90 minutes au reflux, puis est laissée à température ambiante sous agitation une nuit. Le précipité est filtré pour conduire au produit du titre.

### Point de fusion: 118°C

### Microanalyse élémentaire :

| | *C* | *H* | *N* |
|---|---|---|---|
| *% théorique* | *61,36* | *4,58* | *15,90* |
| *% expérimental* | *61,44* | *4,55* | *15,82* |

### Préparation 2 : 3-(1,3-oxazol-2-yl)phénol

### Stade A : 2-(3-méthoxyphényl)-1,3-oxazole

Une suspension contenant 1,16 ml de 1-bromo-3-méthoxybenzène (9,2 mmol), 2,12 ml de 2-(tributylstannanyl)-1,3-oxazole (5,9 mmol), et 276 mg (0,2 mmol) de Palladium tetrakis[triphénylphosphine] dans 50 ml de toluène est agitée une nuit à reflux sous argon. Le toluène est évaporé au rotavapor, le résidu est ensuite repris en suspension dans l'acétate d'éthyle. Après filtration de la suspension, le filtrat est évaporé à sec et le brut est purifié par chromatographie sur gel de silice en éluant avec CH₂Cl₂ puis CH₂Cl₂/acétone 98/2 pour conduire au produit du titre sous forme d'une huile.

### Stade B : 3-(1,3-oxazol-2-yl)phénol

Le produit obtenu au Stade A précédent (250 mg, 1,13 mmol) est mis en suspension dans 10 ml d'une solution aqueuse de HBr 48 % et le milieu réactionnel est agité une nuit à 115 °C. Après refroidissement à température ambiante, le mélange est versé dans 10 ml d'une solution de NaHCO₃ 10 %. Le pH est ajusté à 7 et la phase aqueuse est extraite par de l'acétate d'éthyle. La phase organique est lavée (NaCl saturé), séchée (MgSO₄) pour conduire après filtration et évaporation au produit du titre sous forme d'un solide blanc.

### Préparation 3 : Acide [4-(5-méthyl-1,2,4-oxadiazol-3-yl)phényl]boronique

### Stade A : N'-hydroxy-4-iodobenzenecarboximidamide

Dans 80 ml de DMSO, sont ajoutés 17,3 g (249 mmol) de chlorhydrate d'hydroxylamine puis 35 ml (251 mmol) de triéthylamine. Il se forme un précipité blanc important et le milieu réactionnel est dilué dans un peu de THF. Après 1 heure d'agitation, le précipité est filtré. Le filtrat est récupéré puis transféré dans un ballon auquel sont rajoutés 9,55 g (41,7 mmol) de 4-iodobenzonitrile. La réaction est agitée une nuit à température ambiante puis est refroidie dans un bain de glace et 200 mL d'eau sont ajoutés. Le précipité est filtré pour conduire au produit attendu.

### Microanalyse élémentaire :

| | *C* | *H* | *N* | *I* |
|---|---|---|---|---|
| *% théorique* | *32, 08* | *2, 69* | *10, 69* | *48,43* |
| *% expérimental* | *32,05* | *2,77* | *10,55* | *47,46* |

### Stade B : N'-(acétyloxy)-4-iodobenzènecarboximidamide

A une suspension du produit obtenu au Stade A précédent (8,43 g, 32,17mmol) dans 200 ml de CH₂Cl₂, sont ajoutés 6,72 ml (48,2 mmol) de triéthylamine. 3,34 ml (35,4 mmol) d'anhydride acétique sont additionnés goutte à goutte et la solution est agitée la nuit à température ambiante. Après 3 lavages à l'eau puis à la saumure, la phase organique est séchée sur MgSO₄ et évaporée. Le solide obtenu est repris dans de l'éther isopropylique puis filtré pour conduire au produit du titre

### Point de fusion : 139 °C

### Stade C : 3-(4-iodophényl)-5-méthyl-1,2,4-oxadiazole

A une solution du produit obtenu au Stade B précédent (9,7 g, 31,9 mmol) dans 300 ml de toluène sont ajoutés 150 mg d'acide para-toluène sulfonique et le milieu réactionnel est chauffé au reflux avec un montage dean-stark pendant 24 heures. Le mélange est évaporé à sec et séché à la pompe à vide.

### Point de fusion : 91 °C

### Stade D : 5-méthyl-3-[4-(4,4,5,5-tétraméthyl-1,3,2-dioxaborolan-2-yl)phényl]-1,2,4-oxadiazole

A une solution du produit obtenu au Stade C précédent (500 mg, 1,74 mmol) dans 5 ml de DMF, sont ajoutés 510 mg (5,2 mmol) d'acétate de potassium et 577 mg (2,28 mmol) de bis-pinacolatoborane. Le milieu réactionnel est dégazé 30 minutes avec de l'azote puis 20 mg (0,09 mmol) de Pd(OAc)₂ sont additionnés. La réaction est chauffée 3 heures à 85 °C. Après refroidissement à température ambiante puis addition d'eau, le milieu réactionnel est extrait par de l'acétate d'éthyle. Les phases organiques sont jointes, lavées (NaCl saturée) et séchées (MgSO₄). Le brut est purifié par chromatographie sur gel de silice en éluant avec un mélange heptane/acétate d'éthyle 7/3 pour conduire au produit attendu.

### Point de fusion : 128 °C

### Stade E : Acide [4-(5-méthyl-1,2,4-oxadiazol-3-yl)phényl]boronique

Une suspension du produit obtenu au Stade D précédent (6,5 g, 22,7 mmol) dans 230 ml d'acétone est agitée 24 heures en présence de 18,4 g (86,0 mmol) de méta-périodate de sodium et 70 ml d'une solution aqueuse d'acétate d'ammonium 1 M. L'acétone est évaporé au rotavapor et, après addition d'eau, le milieu réactionnel est extrait avec de l'acétate d'éthyle. Les phases organiques sont jointes, lavées (NaCl saturée) et séchées (MgSO₄) pour conduire après évaporation au produit attendu.

### Préparation 4 : Acide [3-(5-méthyl-1,3,4-oxadiazol-2-yl)phényl]boronique

### Stade A : N'-acétyl-3-iodobenzohydrazide

A une suspension d'acide 3-iodobenzoïque (2,0 g, 8,06 mmol) dans 60 ml de THF, sont rajoutés successivement la DIPEA (1,83 ml, 10,48 mmol) et le TBTU (2,59 g, 8,06 mmol). La réaction est agitée une nuit à température ambiante. On ajoute 1,19 g (16,12 mmol) d'acétylhydrazide et le milieu réactionnel est chauffé 30 heures à reflux. Après évaporation à sec, le résidu est repris dans de l'eau avec un peu de CH₂Cl₂, il se forme un précipité épais qui est filtré pour conduire au produit attendu.

### Point de fusion : 178 °C

### Stade B : 2-(3-iodophényl)-5-méthyl-1, 3, 4-oxadiazole

Le produit obtenu au Stade A précédent (2,9 g, 9,54 mmol) est chauffé à 120 °C pendant 3 heures dans 35 ml de POCl₃. Après évaporation du POCl₃, le résidu est repris dans du toluène et le mélange est à nouveau évaporé à sec. Le résidu d'évaporation est dissous dans l'acétate d'éthyle, la phase organique est lavée successivement par une solution de NaHCO₃ 10 %, par de l'eau puis de la saumure. La phase organique est séchée sur MgSO₄ pour conduire après évaporation au produit attendu sous forme d'un solide blanc crème.

### Point de fusion : 115 °C

### Stade C : 2-méthyl-5-[3-(4,4,5,5-tétraméthyl-1,3,2-dioxaborolan-2-yl)phényl]-1,3,4-oxadiazole

Le produit du titre est obtenu selon le procédé décrit dans le Stade D de la Préparation 3 en prenant comme produit de départ le composé décrit dans le Stade B précédent à la place de la 3-(4-iodophényl)-5-méthyl-1,2,4-oxadiazole.

### Point de fusion : 153 °C

### Stade D : Acide [3-(5-méthyl-1,3,4-oxadiazol-2-yl)phényl]boronique

Le produit du titre est obtenu selon le procédé décrit dans le Stade E de la Préparation 3 en prenant comme produit de départ le composé décrit dans le Stade C précédent à la place de la 5-méthyl-3-[4-(4,4,5,5-tétraméthyl-1,3,2-dioxaborolan-2-yl)phényl]-1,2,4-oxadiazole.

### Point de fusion : 254 °C

### EXEMPLE 1 : 8-[3-(5-méthyl-1,2,4-oxadiazol-3-yl)phénoxyl-3,4-dihydro-2H-5,1,2-benzoxathiazépine 1,1-dioxyde

### Stade A : Chlorure de 5-bromo-2-méthoxybenzènesulfonyle

A l'acide chlorosulfonique (140 mL, 2 mol), refroidi à 0-5°C, sont additionnés goutte à goutte 35 ml (0,279 mol) de 4-bromoanisole. La solution est agitée 30 minutes à 0 °C puis environ 16 heures à température ambiante. Le contenu est lentement versé sur de la glace, puis est encore agité quelques minutes. Le précipité blanc formé est filtré puis abondamment lavé à l'eau.

### Point de fusion : 107 °C

### Stade B : 5-bromo-N-(2-chloroéthyl)-2-méthoxybenzènesulfonamide

A une suspension de 13,4 g (0,115 mol) de chlorhydrate de 2-chloroéthylamine dans 150 ml de CH₂Cl₂, sont ajoutés 34 ml (0,241 mol) de triéthylamine puis goutte à goutte 30 g (0,105 mol) du produit obtenu au Stade A précédent dissous dans 200 ml de CH₂Cl₂. La solution est agitée pendant 2 heures à température ambiante puis est ensuite lavée avec une solution de HCl 1 N, de l'eau et de la saumure. La phase organique est séchée sur MgSO₄ et évaporée. Le solide est repris en suspension dans de l'éther éthylique et filtré pour conduire au produit du titre.

### Point de fusion : 150 °C

### Stade C : 5-bromo-N-(2-chloroéthyl)-2-hydroxybenzènesulfonamide

A une suspension de 29,9 g (91 mmol) du produit obtenu au Stade B précédent dans 450 ml de CH₂Cl₂, sont additionnés goutte à goutte, à température ambiante, 200 mL (200 mmol) d'une solution de BBr₃ 1 M dans le CH₂Cl₂. Après 30 minutes d'agitation, la solution est versée sur un mélange glace/eau tout en maintenant l'agitation. Après décantation et une seule extraction avec CH₂Cl₂, la phase organique est lavée à la saumure, séchée sur MgSO₄ et évaporée. Le résidu huileux est trituré dans l'heptane jusqu'à cristallisation. Le produit du titre est récupéré par filtration.

### Point de fusion : 109 °C

### Stade D : [(5-bromo-2-hydroxyphényl)sulfonyl](2-chloroéthyl)carbamate de tert-butyle

A une solution contenant 25,55 g (81,2 mmol) du produit obtenu au Stade C précédent, 17,72 g (81,2 mmol) de dicarbonate de di-tert-butyle et 496 mg (4,06 mmol) de diméthylaminopyridine dans 500 mL de CH₂Cl₂, sont additionnés goutte à goutte 11,32 g (81,2 mmol) de triéthylamine en solution dans 200 ml de CH₂Cl₂. Le mélange est agité une nuit à température ambiante. La phase organique est lavée (NaCl saturée), séchée (MgSO₄) et évaporé au rotavapor pour conduire au produit du titre.

### Stade E : 8-bromo-3,4-dihydro-2H-5,1,2-benzoxathiazépine 1,1-dioxyde

37 g (81 mmol) du produit obtenu au Stade D précédent sont chauffés au reflux en présence de 2,69 g (16 mmol) de KI et 33,6 g (240 mmol) de K₂CO₃ dans 1,2 L d'éthanol pendant environ 18 heures. Après refroidissement, les sels sont filtrés et rincés à l'acétone. Après évaporation, le filtrat est repris dans de l'eau. Le mélange est acidifié avec une solution de HCl 1 N puis extrait 3 fois avec CH₂Cl₂. La phase organique est lavée 2 fois à la saumure, séchée sur MgSO₄ et évaporée. Le résidu obtenu (~ 22 g) est ensuite trituré dans l'heptane puis le mélange est laissé décanter afin d'éliminer l'heptane surnageant. Le résidu est une nouvelle fois trituré dans un mélange heptane/éther isopropylique pour obtenir un solide. Après filtration, le produit est rapidement séché puis broyé au mortier.

Le solide obtenu est mis en solution dans de l'heptane, agité pendant environ 1 heure et filtré pour conduire au produit attendu.

### Point de fusion : 129 °C

### Stade F : 8-bromo-3,4-dihydro-2H-5,1,2-benzoxathiazépine-2-carboxylate 1,1 - dioxyde de tert-butyle

A une solution de 19,65 g (70,6 mmol) du produit obtenu au Stade E précédent et 259 mg (2 mmol) de DMAP dans 200 ml de CH₂Cl₂, sont additionnés goutte à goutte 23,1 g (106 mmol) de dicarbonate de di-*tert*-butyle en solution dans 125 ml de CH₂Cl₂. Le mélange réactionnel est agité à température ambiante puis, au bout de 3 heures, 100 mg de DMAP sont ajoutés. La solution est agitée pendant 45 minutes. Après 2 lavages à l'eau et à la saumure, la phase organique est séchée sur MgSO₄ et évaporée. Le résidu d'évaporation est trituré dans l'heptane. Le solide obtenu est mis en solution dans de l'heptane, agité pendant 48 heures donnant un précipité qui sera filtré pour conduire au produit du titre.

### Point de fusion : 119 °C

### Stade G : Acide [2-(tert-butoxycarbonyl)-1,1-dioxydo-3,4-dihydro-2H-5,1,2-benzoxathiazépin-8-yl]boronique

A une solution du produit obtenu au Stade F précédent (21,6 g, 57,3 mmol) et de 40 ml de tri-isopropylborate dans 210 ml de THF, refroidie à -65 °C et sous N₂, sont additionnés goutte à goutte 90 ml (143 mmol) d'une solution de *n*BuLi 1,6 M. Le mélange réactionnel est agité 1 heure à -65 °C et laissé revenir à température ambiante. Après 1 heure d'agitation, la solution est refroidie dans un bain de glace et hydrolysée par addition de 320 ml d'une solution de HCl 0,5 M. Après 3 extractions au CH₂Cl₂, la phase organique est lavée à la saumure, séchée sur MgSO₄ et évaporée. Le résidu est repris dans de l'heptane qui, après évaporation, est trituré dans l'heptane et filtré pour conduire au produit du titre.

### Point de fusion : 197 °C

### Stade H : 8-[3-(5-méthyl-1,2,4-oxadiazol-3-yl)phénoxy]-3,4-dihydro-2H-5,1,2-benzoxathiazépine-2-carboxylate 1,1-dioxyde de tert-butyle

Une suspension contenant 2,5 g (7,28 mmol) du produit obtenu au Stade G précédent, 1,17 g (6,62 mmol) du composé de la Préparation 1, 1,61 ml (19,9 mmol) de pyridine, 1,8 g (9,9 mmol) d'acétate de cuivre et de 8 g de tamis 4 Å dans 125 ml de CH₂Cl₂ est agitée à température ambiante et à l'air pendant 20 heures. Le tamis est filtré et le filtrat est rincé avec 125 ml d'un mélange CH₂Cl₂/MeOH. Ensuite, 6 g de SiO₂ sont ajoutés au filtrat avant évaporation à sec. Le brut est purifié par chromatographie sur gel de silice en éluant avec un mélange CH₂Cl₂/acétone 97 / 3 pour conduire au produit attendu sous forme de meringue.

### Stade I: 8-[3-(5-méthyl-1,2,4-oxadiazol-3-yl)phénoxy]-3,4-dihydro-2H-5,1,2-benzoxathiazépine 1,1-dioxyde

Une solution de 1,92 g (4,05 mmol) du produit obtenu au Stade H précédent est chauffée à 80°C pendant 2 heures dans 15 ml d'une solution de HCl 4 N dans le dioxane. Le mélange est évaporé à sec et séché sous vide. Le brut est purifié par chromatographie sur gel de silice en éluant avec un mélange CH₂Cl₂/AcOEt. Le solide repris par de l'éther isopropylique et filtré pour conduire au produit du titre

### Point de fusion : 177 °C

### EXEMPLE 2: 8-[3-(1,3-thiazol-2-yl)phénoxy]-3,4-dihydro-2H-5,1,2-benzoxathiazépine 1,1-dioxyde

### Stade A : 8-[3-(1,3-thiazol-2-yl)phénoxy]-3,4-dihydro-2H-5,1,2-benzoxathiazépine -2-carboxylate 1,1-dioxyde de tert-butyle

Le produit du titre est obtenu selon le protocole décrit dans le Stade H de l'Exemple 1 en utilisant le (3-thiazol-2-yl)phénol (dont la préparation est décrite dans Bioorg. Med. Chem. 2003, 11(7), 1235-48) à la place du composé de la Préparation 1.

### Stade B : 8-[3-(1,3-thiazol-2-yl)phénoxy]-3, 4-dihydro-2H-5,1,2-benzoxathiazépine 1,1-dioxyde

Le produit du titre est obtenu selon le protocole décrit dans le Stade I de l'Exemple 1 en utilisant le composé obtenu au Stade A précédent à la place du 8-[3-(5-méthyl-1,2,4-oxadiazol-3-yl)phénoxy]-3,4-dihydro-2*H*-5,1,2-benzoxathiazépine-2-carboxylate 1,1-dioxyde de *tert*-butyle.

### Point de fusion : 148°C

### Microanalyse élémentaire :

| | *C* | *H* | *N* | *S* |
|---|---|---|---|---|
| *% théorique* | *54,53* | *3,77* | *7,48* | *17,13* |
| *% expérimental* | *54, 68* | *3,85* | *7,34* | *17,18* |

### EXEMPLE 3 : 8-[3-(1,3-thiazol-5-yl)phénoxy]-3,4-dihydro-2H-5,1,2-benzoxathiazépine 1,1-dioxyde

### Stade A : 8-[3-(1,3-thiazol-5-yl)phénoxy]-3,4-dihydro-2H-5,1,2-benzoxathiazépine -2-carboxylate 1,1-dioxyde de tert-butyle

Le produit du titre est obtenu selon le protocole décrit dans le Stade H de l'Exemple 1 en utilisant le (3-thiazol-5-yl)phénol (dont la préparation est décrite dans Bioorg. Med. Chem. 2003, 11(7), 1235-48) à la place du composé de la Préparation 1.

### Stade B : 8-[3-(1,3-thiazol-5-yl)phénoxy]-3,4-dihydro-2H-5,1,2-benzoxathiazépine 1,1-dioxyde

Le produit du titre est obtenu selon le protocole décrit dans le Stade I de l'Exemple 1 en utilisant le composé obtenu au Stade A précédent à la place du 8-[3-(5-méthyl-1,2,4-oxadiazol-3-yl)phénoxy]-3,4-dihydro-2*H*-5,1,2-benzoxathiazépine-2-carboxylate 1,1-dioxyde de *tert-*butyle.

### Point de fusion : 60°C (meringue)

### Microanalyse élémentaire :

| | *C* | *H* | *N* | *S* |
|---|---|---|---|---|
| *% théorique* | *54,53* | *3,77* | *7,48* | *17,13* |
| *% expérimental* | *54, 69* | *3,76* | *7, 06* | *17,10* |

### EXEMPLE 4 : 8-[3-(1,3-oxazol-2-yl)phénoxy]-3,4-dihydro-2H-5,1,2-benzoxathiazépine 1,1-dioxyde

### Stade A : 8-[3-(1,3-oxazol-2-yl)phénoxy]-3,4-dihydro-2H-5,1,2-benzoxathiazépine-2-carboxylate 1,1-dioxyde de tert-butyle

Le produit du titre est obtenu selon le protocole décrit dans le Stade H de l'Exemple 1 en utilisant le composé de la Préparation 2 à la place du composé de la Préparation 1.

### Stade B : 8-[3-(1,3-oxazol-2-yl)phénoxy]-3,4-dihydro-2H-5,1,2-benzoxathiazépine 1,1-dioxyde

Le produit du titre est obtenu selon le protocole décrit dans le Stade I de l'Exemple 1 en utilisant le composé obtenu au Stade A précédent à la place du 8-[3-(5-méthyl-1,2,4-oxadiazol-3-yl)phénoxy]-3,4-dihydro-2*H*-5,1,2-benzoxathiazépine-2-carboxylate 1,1-dioxyde de *tert*-butyle.

### Point de fusion : 180 °C

### Microanalyse élémentaire :

| | *C* | *H* | *N* | *S* |
|---|---|---|---|---|
| *% théorique* | *56,98* | *3,94* | *7,82* | *8,95* |
| *% expérimental* | *56, 60* | *3,92* | *7, 65* | *8, 61* |

### EXEMPLE 5 : 8-[4-(5-méthyl-1,2,4-oxadiazol-3-yl)phénoxy]-3,4-dihydro-2H-5,1,2-benzoxathiazépine 1,1-dioxyde

### Stade A : 8-hydroxy-3,4-dihydro-2H-5,1,2-benzoxathiazépine-2-carboxylate 1,1-dioxyde de tert-butyle

Une suspension de 4,7 g (13,69 mmol) du produit du Stade G de l'Exemple 1 dans un mélange de 90 ml de THF et 90 ml d'eau est agitée à température ambiante en présence de 5,27 g (34 mmol) de perborate de sodium tétrahydrate pendant une nuit. Le milieu réactionnel est dilué par addition d'une solution aqueuse saturée en NH₄Cl puis est extrait par CH₂Cl₂. Les phases organiques sont jointes, lavées (NaCl saturé), séchées (MgSO4) et évaporées au rotavapor. Le résidu d'évaporation est repris et trituré dans l'heptane en présence de quelques goutte d'éther isopropylique, il se forme un précipité qui est filtré pour conduire au produit attendu.

### Point de fusion : 143 °C

### Stade B : 8-[4-(5-méthyl-1,2,4-oxadiazol-3-yl)phénoxy]-3,4-dihydro-2H-5,1,2-benzoxathiazépine-2-carboxylate 1,1-dioxyde de tert-butyle

Le produit du titre est obtenu selon le protocole décrit dans le Stade H de l'Exemple 1 en utilisant le composé de la Préparation 3 à la place du composé de la Préparation 1.

### Stade C : 8-[4-(5-méthyl-1,2,4-oxadiazol-3-yl)phénoxy]-3,4-dihydro-2H-5,1,2-benzoxathiazépine 1,1-dioxyde

Le produit du titre est obtenu selon le protocole décrit dans le Stade I de l'Exemple 1 en utilisant le composé obtenu au Stade B précédent à la place du 8-[3-(5-méthyl-1,2,4-oxadiazol-3-yl)phénoxy]-3,4-dihydro-2*H*-5,1,2-benzoxathiazépine-2-carboxylate 1,1-dioxyde de *tert*-butyle.

### Point de fusion : 171 °C

### Microanalyse élémentaire :

| | *C* | *H* | *N* | *S* |
|---|---|---|---|---|
| *% théorique* | *54, 69* | *4,05* | *11,25* | *8,59* |
| *% expérimental* | *54,81* | *4, 07* | *10,96* | *8,54* |

### EXEMPLE 6 : 8-[3-(5-méthyl-1,3,4-oxadiazol-2-yl)phénoxy]-3,4-dihydro-2H-5,1,2-benzoxathiazépine 1,1-dioxyde

### Stade A : 8-[3-(5-méthyl-1,3,4-oxadiazol-2-yl)phénoxy]-3,4-dihydro-2H-5,1,2-benzoxathiazépine-2-carboxylate 1,1-dioxyde de tert-butyle

Le produit du titre est obtenu selon le protocole décrit dans le Stade H de l'Exemple 1 en faisant réagir le produit obtenu au Stade A de l'Exemple 5 avec le composé de la Préparation 4 à la place du composé de la Préparation 1.

### Stade B : 8-[3-(5-méthyl-1,3,4-oxadiazol-2 yl)phénoxy]-3, 4-dihydro-2H-5,1,2-benzoxathiazépine 1,1-dioxyde

Le produit du titre est obtenu selon le protocole décrit dans le Stade I de l'Exemple 1 en utilisant le composé obtenu au Stade A précédent à la place du 8-[3-(5-méthyl-1,2,4-oxadiazol-3-yl)phénoxy]-3,4-dihydro-2*H*-5,1,2-benzoxathiazépine-2-carboxylate 1,1-dioxyde de *tert*-butyle.

### Point de fusion : 192 °C

### EXEMPLE 7 : 3-[(1,1-dioxydo-3,4-dihydro-2H-5,1,2-benzoxathiazépin-8-yl)oxy] benzoate d'éthyle

### Stade A : 8-[3-(éthoxycarbonyl)phénoxy]-3,4-dihydro-2H-5,1,2-benzoxathiazépine-2-carboxylate 1,1-dioxyde de tert-butyle

Le produit du titre est obtenu selon le protocole décrit dans le Stade H de l'Exemple 1 en faisant réagir le produit obtenu au Stade A de l'Exemple 5 avec l'acide [3-(éthoxycarbonyl)phényl]boronique.

### Stade B : 3-[(1,1-dioxydo-3,4-dihydro-2H-5,1,2-benzoxathiazépin-8-yl)oxy] benzoate d'éthyle

Le produit du titre est obtenu selon le protocole décrit dans le Stade I de l'Exemple 1 en utilisant le composé obtenu au Stade A précédent à la place du 8-[3-(5-méthyl-1,2,4-oxadiazol-3-yl)phénoxy]-3,4-dihydro-2*H*-5,1,2-benzoxathiazépine-2-carboxylate 1,1-dioxyde de *tert*-butyle.

### Point de fusion : 120°C

### Microanalyse élémentaire:

| | *C* | *H* | *N* | *S* |
|---|---|---|---|---|
| *% théorique* | *56,19* | *4,72* | *3,85* | *8,82* |
| *% expérimental* | *55,71* | *4,70* | *4, 07* | *9, 02* |

### EXEMPLE 8 : 3-[(1,1-dioxydo-3,4-dihydro-2H-5,1,2-benzoxathiazépin-8-yl)oxy] benzonitrile

### Stade A : 8-(3-cyanophénoxy)-3,4-dihydro-2H-5,1,2-benzoxathiazépine-2-carboxylate 1,1-dioxyde de tert-butyle

Le produit du titre est obtenu selon le protocole décrit dans le Stade H de l'Exemple 1 en faisant réagir le produit obtenu au Stade A de l'Exemple 5 avec l'acide (3-cyanophényl)boronique.

### Stade B : 3-[(1,1-dioxydo-3,4-dihydro-2H-5,1,2-benzoxathiazépin-8-yl)oxy] benzonitrile

Le produit du titre est obtenu selon le protocole décrit dans le Stade I de l'Exemple 1 en utilisant le composé obtenu au Stade A précédent à la place du 8-[3-(5-méthyl-1,2,4-oxadiazol-3-yl)phénoxy]-3,4-dihydro-2*H*-5,1,2-benzoxathiazépine-2-carboxylate 1,1-dioxyde de *tert*-butyle.

### Point de fusion : 131 °C

### Microanalyse élémentaire :

| | *C* | *H* | *N* | *S* |
|---|---|---|---|---|
| *% théorique* | *56,95* | *3,82* | *8,86* | *10,14* |
| *% expérimental* | *56, 66* | *3,88* | *8, 64* | *9,98* |

### EXEMPLE 9 : N-{3-[(1,1-dioxydo-3,4-dihydro-2H-5,1,2-benzoxathiazépin-8-yl)oxy] benzyl}méthanesulfonamide

### Stade A : 8-(3-{[(méthylsulfonyl)amino]méthyl)phénoxy}-3,4-dihydro-2H-5,1,2-benzoxathiazépine-2-carboxylate 1,1-dioxyde de tert-butyle

Le produit du titre est obtenu selon le protocole décrit dans le Stade H de l'Exemple 1 en faisant réagir le produit obtenu au Stade A de l'Exemple 5 avec l'acide (3-{[(méthylsulfonyl)amino]méthyl}phényl)boronique.

### Stade B : N-{3-[(1,1-dioxydo-3,4-dihydro-2H-5,1,2-benzoxathiazépin-8-yl)oxy] benzyl}méthanesulfonamide

Le produit du titre est obtenu selon le protocole décrit dans le Stade I de l'Exemple 1 en utilisant le composé obtenu au Stade A précédent à la place du 8-[3-(5-méthyl-1,2,4-oxadiazol-3-yl)phénoxy]-3,4-dihydro-2*H*-5,1,2-benzoxathiazépine-2-carboxylate 1,1-dioxyde de *tert*-butyle.

### Point de fusion : 57-60 °C

### EXEMPLE 10 : 8-phénoxy-3,4-dihydro-2H-5,1,2-benzoxathiazépine 1,1-dioxyde

### Stade A : 8-phénoxy-3,4-dihydro-2H-5,1,2-benzoxathiazépine-2-carboxylate 1,1-dioxyde de tert-butyle

Le produit du titre est obtenu selon le protocole décrit dans le Stade H de l'Exemple 1 en faisant réagir le produit obtenu au Stade A de l'Exemple 5 avec l'acide phénylboronique.

### Stade B : 8-phénoxy-3,4-dihydro-2H-5,1,2-benzoxathiazépine 1,1-dioxide

Le produit du titre est obtenu selon le protocole décrit dans le Stade I de l'Exemple 1 en utilisant le composé obtenu au Stade A précédent à la place du 8-[3-(5-méthyl-1,2,4-oxadiazol-3-yl)phénoxy]-3,4-dihydro-2*H*-5,1,2-benzoxathiazépine-2-carboxylate 1,1-dioxyde de *tert*-butyle.

### Point de fusion : 110 °C

### EXEMPLE 11 : 3-[(1,1-dioxydo-3,4-dihydro-2H-5,1,2-benzoxathiazépin-8-yl)oxy]-N'-hydroxybenzènecarboximidamide

Le produit du titre est obtenu selon le protocole décrit dans le Stade A de la Préparation 1 en utilisant le composé de l'Exemple 8 à la place de la 3-hydroxybenzonitrile.

### Point de fusion : 163-166 °C

### Microanalyse élémentaire :

| | *C* | *H* | *N* | *S* |
|---|---|---|---|---|
| *% théorique* | *51,57* | *4,33* | *12, 03* | *9,18* |
| *% expérimental* | *51, 27* | *4,42* | *11, 62* | *9,31* |

### EXEMPLE 12 : 8-{3-[5-(trifluorométhyl)-1,2,4-oxadiazol-3-yl]phénoxy}-3,4-dihydro-2H-5,1,2-benzoxathiazépine 1,1-dioxyde

A une suspension du composé de l'Exemple 11 (800 mg, 2,29 mmol) dans 15 ml de CH₂Cl₂, sont ajoutés 958 µl (6,87 mmol) de triéthylamine puis 701 µl (5,04 mmol) d'anhydride trifluoroacétique au goutte à goutte. Après 1 heure d'agitation à température ambiante, le milieu réactionnel est lavé par de l'eau puis par une solution saturée en NaCl et séché sur MgSO₄. Le brut est purifié par chromatographie sur gel de silice en éluant par du CH₂Cl₂ pour conduire au produit attendu sous forme de meringue.

### Microanalyse élémentaire :

| | *C* | *H* | *N* | *S* |
|---|---|---|---|---|
| *% théorique* | *47,78* | *2,83* | *9,83* | *7,50* |
| *% expérimental* | *47, 66* | *3,18* | *9,59* | *7,69* |

### EXEMPLE 13 : 8-[3-(3-méthyl-1,2,4-oxadiazol-5-yl)phénoxy]-3,4-dihydro-2H-5,1,2-benzoxathiazépine 1,1-dioxyde

### Stade A : Acide 3-[(1,1-dioxydo-3,4-dihydro-2H-5,1,2-benzoxathiazépin-8-yl)oxy]benzoïque

Une suspension de 1,0 g (2,23 mmol) du produit obtenu au Stade A de l'Exemple 7 dans 20 ml d'une solution de NaOH 1 N et 1 ml de THF est agitée 3 heures à 100 °C. Après refroidissement à température ambiante, le milieu réactionnel est acidifié par addition lente d'une solution de HCl 1 N. Le précipité blanc est filtré et séché pour conduire au produit attendu.

### Stade B : 3-[(1,1-dioxydo-3,4-dihydro-2H-5,1,2-benzoxathiazépin-8-yl)oxy]-N-[(1Z)-N-hydroxyéthanimidoyl]benzamide

A une suspension du produit obtenu au Stade A précédent (540 mg, 1,61 mmol) dans 10 ml de CH₂Cl₂, sont ajoutés successivement 516 mg (1,61 mmol) de TBTU puis 364 µl (2,09 mmol) de DIEA. Après 20 minutes d'agitation, 120 mg (1,61 mmol) de N-hydroxyacétamidine sont ajoutés et le milieu réactionnel est agité 1,5 heures à température ambiante. Le milieu réactionnel est lavé par de l'eau puis par une solution saturée en NaCl, séché (MgSO₄) et évaporé à sec. Le résidu est repris dans du CH₂Cl₂, il se forme un précipité blanc qui est filtré pour conduire au produit attendu.

### Stade C : 8-[3-(3-méthyl-1,2,4-oxadiazol-5-yl)phénoxy]-3,4-dihydro-2H-5,1,2-benzoxathiazépine 1,1-dioxyde

A une solution de 390 mg (0,99 mmol) du produit obtenu au Stade B précédent dans 50 ml de toluène, sont ajoutés 35 mg d'acide para-toluène sulfonique et le milieu réactionnel est chauffé à reflux avec un système dean-stark pendant 12 heures. Après évaporation, le brut réactionnel est purifié sur gel de silice en éluant avec un mélange CH₂Cl₂/AcOEt 9 / 1 pour conduire au produit attendu.

### Point de fusion : 140 °C

### Microanalyse élémentaire :

| | *C* | *H* | *N* | *S* |
|---|---|---|---|---|
| *% théorique* | *54,69* | *4,05* | *11,25* | *8,59* |
| *% expérimental* | *54,78* | *4,11* | *11,00* | *8,70* |

### ETUDE PHARMACOLOGIQUE

### EXEMPLE A : Etude de l'effet des produits sur la dépolarisation membranaire induite par l'AMPA sur des cultures primaires de neurones de rat.

Le test consiste en la mesure *in vitro,* par fluorescence, de la dépolarisation membranaire induite sur des neurones embryonnaires de rat en culture, par l'action conjointe de l'AMPA et du produit testé, en comparaison à l'action de l'AMPA seul. Les cellules du cerveau sont mises en culture et maintenues dans un incubateur de culture cellulaire pendant 18 jours. Après incubation, le milieu de culture est retiré et remplacé par du milieu de chargement en sonde de fluorescence pour mesure du potentiel membranaire (20 µl ; kit de potentiel de membrane de Molecular Devices) et laissées à température ambiante pendant 1 heure. La fluorescence de base des puits est lue (appareil FDSS de Hamamatsu), puis l'AMPA est injecté sur les cellules (20 µl ; gamme de concentration de 3 à 100 µM) et l'action de l'AMPA est mesurée en cinétique. Le produit testé est ensuite introduit dans les puits (20 µl ; en gamme de concentration, croisée avec celle de l'AMPA) et l'action du produit est mesurée en cinétique. A l'issue de chacune des 2 périodes de mesure en cinétique, la valeur retenue pour chaque puits est la moyenne de la lecture sur les 15 dernières secondes de la période. On représente les courbes d'effet de l'AMPA aux différentes concentrations de produit. Pour chaque concentration de produit, la valeur retenue est l'aire sous la courbe d'AMPA à cette concentration et l'EC_{2X}, concentration de produit qui double le potentiel membranaire induit par l'AMPA, est calculée.

Les composés de l'invention potentialisent fortement les effets excitateurs de l'AMPA. A titre d'exemple, les composés de l'Exemple 1 et de l'Exemple 13 présentent une EC_{2X} de 5 et 18 µM, respectivement.

### EXEMPLE B : Reconnaissance d'objet chez la souris CD1

Le test de reconnaissance d'objet (Behav. Brain Res., 1988, 31, 47-59) est basé sur l'activité exploratoire spontanée de l'animal et possède les caractéristiques de la mémoire épisodique chez l'Homme. Sensible au vieillissement (Eur. J. Pharm. 1997, 325, 173-180), ainsi qu'aux dysfonctionnements cholinergiques (Pharm. Biochem. Behav., 1996, 53(2), 277-283), ce test de mémoire est fondé sur l'exploration différentielle de 2 objets, l'un familier, l'autre nouveau. La procédure expérimentale, adaptée à la souris CD1, comporte 3 phases qui se déroulent dans la même enceinte expérimentale. Pendant la 1^{ère} phase qui dure 30 minutes, les souris sont habituées à l'environnement (phase d'habituation). Pendant la 2^{e} phase, qui a lieu le lendemain, deux objets identiques sont placés dans l'enceinte et la souris est libre de les explorer (phase de familiarisation). Lorsque cette exploration atteint une durée totale de 20 secondes, la souris est retirée de l'enceinte. Au cours de la 3^{e} phase (5 minutes, phase de rétention), 6 heures plus tard, l'un des deux objets est à nouveau présenté (il acquiert le statut d'objet « familier »), ainsi qu'un nouvel objet (objet « nouveau »). La durée d'exploration, exprimée en secondes, de chacun des deux objets, est chronométrée. Les animaux témoins, ayant reçu préalablement le véhicule par voie orale 60 minutes avant la phase de familiarisation, explorent pendant une durée équivalente l'objet « familier » et l'objet « nouveau » pendant la phase de rétention, ce qui signifie l'oubli de l'objet déjà présenté. Les animaux ayant reçu un composé facilitateur mnémocognitif, explorent de façon préférentielle l'objet nouveau, ce qui signifie le maintien du souvenir de l'objet déjà présenté.

Les résultats obtenus avec les composés de la présente invention montrent une exploration significativement plus importante de l'objet nouveau par rapport à l'objet familier aux doses de 0,3, 1 et 3 mg/kg, PO, en doublant voire triplant la durée d'exploration de l'objet « nouveau » par rapport à l'objet « familier », ce qui indique que les composés de l'invention améliorent la mémorisation de façon importante.

### EXEMPLE C : Composition pharmaceutique

| Formule de préparation pour 1000 comprimés dosés à 10 mg en 8-[3-(5-méthyl-1,2,4- oxadiazol-3-yl)phénoxy]-3,4-dihydro-2*H*-5,1,2-benzoxathiazépine 1,1-dioxyde | |
|---|---|
| (Exemple 1) | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 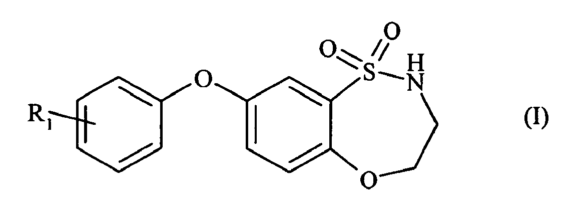 dans laquelle R₁ représente un atome d'hydrogène, un groupement cyano, un groupement alkoxycarbonyle (C₁-C₆) linéaire ou ramifié, un groupement alkyl(C₁-C₆)Sulfonylaminoalkyle (C₁-C₆) linéaire ou ramifié, un groupement N-hydroxycarboximidamide, ou un groupement hétérocyclique,
leurs énantiomères et leurs diastéréoisomères, ainsi que leurs sels d'addition avec un acide ou une base pharmaceutiquement acceptable,
étant entendu que le terme groupement hétérocyclique désigne un groupement aromatique monocyclique à 5 chaînons, contenant un à quatre hétéroatomes, identiques ou différents, choisis parmi azote, oxygène et soufre, ledit groupement hétérocyclique pouvant être éventuellement substitué par un ou plusieurs substituants, identiques ou différents, choisis parmi alkyle (C₁-C₆) linéaire ou ramifié ou polyhalogénoalkyle (C₁-C₆) linéaire ou ramifié.

2. Composés de formule (I) selon la revendication 1, **caractérisés en ce que** R₁ représente un groupement hétérocyclique.

3. Composés de formule (I) selon la revendication 1, **caractérisés en ce que** R₁ représente un groupement hétérocyclique monocyclique aromatique à 5 chaînons contenant au moins un atome d'azote, ledit groupement pouvant être éventuellement substitué par un ou plusieurs substituants, identiques ou différents, choisis parmi alkyle (C₁-C₆) linéaire ou ramifié ou polyhalogénoalkyle (C₁-C₆) linéaire ou ramifié.

4. Composés de formule (I) selon la revendication 1, **caractérisés en ce que** R₁ représente un groupement pyrrolyl, pyrazolyl, imidazolyl, triazolyl, tetrazolyl, isoxazolyl, oxazolyl, isothiazolyl, thiazolyl, thiadiazolyl, dithiazolyl ou oxadiazolyl, chacun des groupements pouvant être éventuellement substitués par un ou plusieurs substituants, identiques ou différents, choisis parmi alkyle (C₁-C₆) linéaire ou ramifié ou polyhalogénoalkyle (C₁-C₆) linéaire ou ramifié.

5. Composés de formule (I) selon la revendication 1, **caractérisés en ce que** R₁ représente un groupement thiazolyl ou oxadiazolyl, chacun des groupements pouvant être éventuellement substitués par un ou plusieurs substituants, identiques ou différents, choisis parmi alkyle (C₁-C₆) linéaire ou ramifié ou polyhalogénoalkyle (C₁-C₆) linéaire ou ramifié.

6. Composés de formule (I) selon la revendication 1, **caractérisés en ce que** R₁ représente un groupement 1,3-thiazolyl ou 1,2,4-oxadiazolyl, chacun des groupements pouvant être éventuellement substitués par un ou plusieurs substituants, identiques ou différents, choisis parmi alkyle (C₁-C₆) linéaire ou ramifié ou polyhalogénoalkyle (C₁-C₆) linéaire ou ramifié.

7. Composés de formule (I) selon la revendication 1, **caractérisés en ce que** R₁ représente un groupement 1,3-thiazolyl ou 1,2,4-oxadiazolyl substitué par un groupement méthyle ou trifluorométhyle.

8. Composés de formule (I) selon la revendication 1, **caractérisés en ce que** R₁ est en position méta du noyau phénoxy qui le porte.

9. Composés de formule (I) selon la revendication 1, qui sont le :
• le 8-[3-(5-méthyl-1,2,4-oxadiazol-3-yl)phénoxy]-3,4-dihydro-2H-5,1,2-benzoxathiazépine 1,1-dioxyde ;
• le 8-[3-(3-méthyl-1,2,4-oxadiazol-5-yl)phénoxy]-3,4-dihydro-2H-5,1,2-benzoxathiazépine 1,1-dioxyde ;
leurs énantiomères, leurs diastéréoisomères ainsi que leurs sels d'addition à un acide ou à une base pharmaceutiquement acceptable.

10. Procédé de préparation des composés de formule (I) selon la revendication 1, **caractérisé en ce que** l'on utilise comme produit de départ un composé de formule (II) : 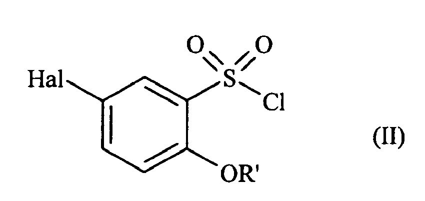 dans laquelle Hal représente un atome d'halogène, tel que le fluor, le chlore ou le brome, et R' représente un groupement alkyle (C₁-C₆) linéaire ou ramifié,
que l'on met en réaction avec de la 2-chloroéthylamine en milieu basique pour conduire au composé de formule (III) : 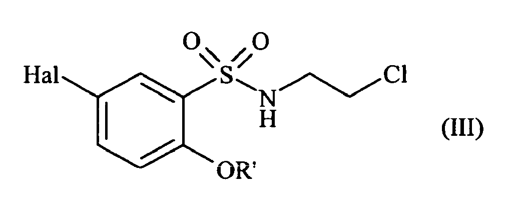 dans laquelle Hal et R' sont tels que définis précédemment,
qui subit alors l'action d'un dérivé borique pour conduire au composé de formule (IV) : 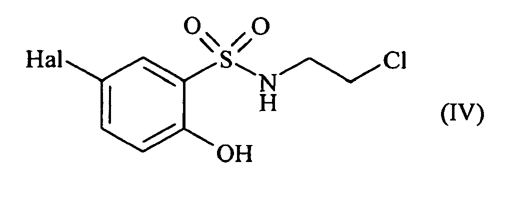 dans laquelle Hal est tel que défini précédemment,
qui est ensuite cyclisé pour conduire au composé de formule (V) : 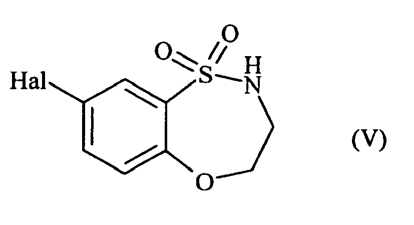 dans laquelle Hal est tel que défini précédemment,
qui subit une réaction de protection sur l'atome d'azote pour conduire au composé de formule (VI) : 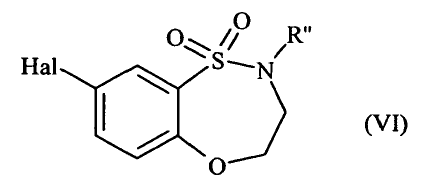 dans laquelle Hal est tel que défini précédemment et R" représente un groupement protecteur de la fonction amine, comme par exemple le groupement *tert-*butyloxycarbonyle,
que l'on transforme en acide boronique pour conduire aux composés de formule (VII) : 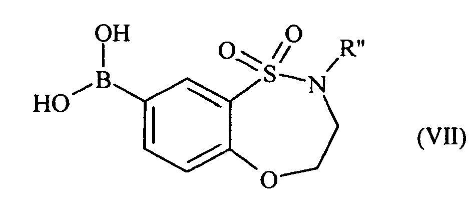 dans laquelle R" est tel que défini précédemment,
que l'on fait réagir avec un alcool de formule (VIII) : 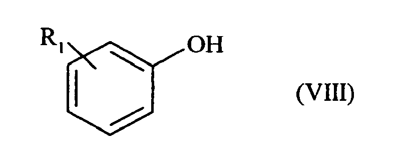 dans laquelle R₁ a la même signification que dans la formule (I),
pour conduire au composé de formule (IX), 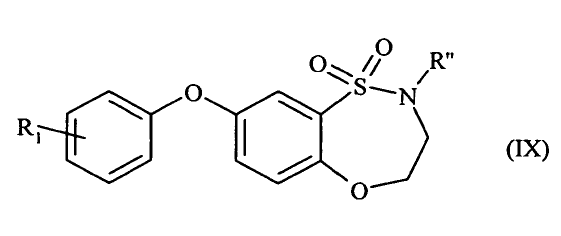 dans laquelle R₁ et R" sont tels que définis précédemment ;
qui est ensuite soumis à une réaction de déprotection de la fonction amine pour conduire au composé de formule (I),
une variante dans la préparation des composés de formule (I) consistant en l'hydrolyse du composé de formule (VII) pour conduire au composé de formule (X) : 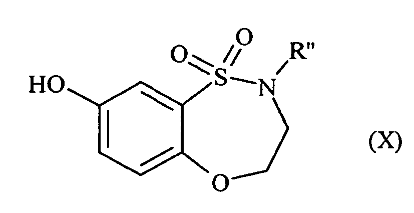 dans laquelle R" est tel que défini précédemment,
que l'on fait réagir avec un dérivé d'acide boronique de formule (XI) : 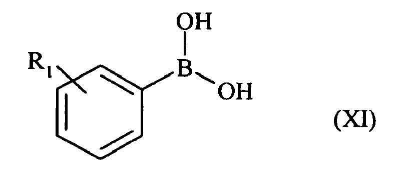 dans laquelle R₁ a la même signification que dans la formule (I),
pour conduire au composé de formule (IX),
qui est ensuite soumis à une réaction de déprotection de la fonction amine pour conduire au composé de formule (I),
une autre alternative dans la préparation des composés de formule (I) consistant, après la préparation des composés de formule (IX), en l'utilisation des réactions classiques de chimie afin de modifier, dans un deuxième temps, le substituant du noyau phénoxy,
composé de formule (I) qui peut être ensuite purifié selon une technique classique de séparation, que l'on transforme, si on le souhaite en ses sels d'addition à un acide ou à une base pharmaceutiquement acceptable et dont on sépare éventuellement les isomères selon une technique classique de séparation.

11. Composés de formule (V) : 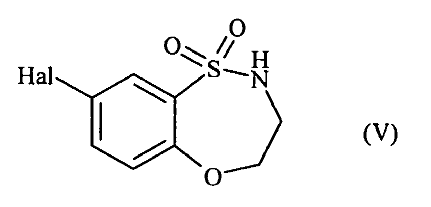 dans laquelle Hal représente un atome d'halogène tel que le fluor, le chlore ou le brome,
**caractérisés en ce qu'**ils sont utiles en tant qu'intermédiaires de synthèse des composés de formule (I).

12. Composition pharmaceutique contenant comme principe actif un composé selon l'une quelconque des revendications 1 à 9, en combinaison avec un ou plusieurs véhicules inertes, non toxiques et pharmaceutiquement acceptables.

13. Compositions pharmaceutiques selon la revendication 12 utiles en tant que modulateurs du récepteur AMPA.

14. Compositions pharmaceutiques selon la revendication 12 utiles pour le traitement ou la prévention des désordres mnémocognitifs associés à l'âge, aux syndromes anxieux ou dépressifs, aux maladies neurodégénératives progressives, à la maladie d'Alzheimer, à la maladie de Parkinson, à la maladie de Pick, à la chorée d'Huntington, à la maladie de Korsakoff, à la schizophrénie, aux séquelles des maladies neurodégénératives aiguës, aux démences frontales et sous-corticales, aux séquelles de l'ischémie et aux séquelles de l'épilepsie.

15. Utilisation des composés de formule (I) selon l'une quelconque des revendications 1 à 9 pour la fabrication de médicaments utiles en tant que modulateurs du récepteur AMPA.

16. Utilisation des composés de formule (I) selon l'une quelconque des revendications 1 à 9 pour la fabrication de médicaments utiles pour le traitement ou la prévention des désordres mnémocognitifs associés à l'âge, aux syndromes anxieux ou dépressifs, aux maladies neurodégénératives progressives, à la maladie d'Alzheimer, à la maladie de Parkinson, à la maladie de Pick, à la chorée d'Huntington, à la maladie de Korsakoff, à la schizophrénie, aux séquelles des maladies neurodégénératives aiguës, aux démences frontales et sous-corticales, aux séquelles de l'ischémie et aux séquelles de l'épilepsie.

17. Composés de formule (I) selon l'une quelconque des revendications 1 à 9 utiles en tant que modulateurs du récepteur AMPA.

18. Composés de formule (I) selon l'une quelconque des revendications 1 à 9 utiles pour le traitement ou la prévention des désordres mnémocognitifs associés à l'âge, aux syndromes anxieux ou dépressifs, aux maladies neurodégénératives progressives, à la maladie d'Alzheimer, à la maladie de Parkinson, à la maladie de Pick, à la chorée d'Huntington, à la maladie de Korsakoff, à la schizophrénie, aux séquelles des maladies neurodégénératives aiguës, aux démences frontales et sous-corticales, aux séquelles de l'ischémie et aux séquelles de l'épilepsie.

## Claims

1. Compounds of formula (I): 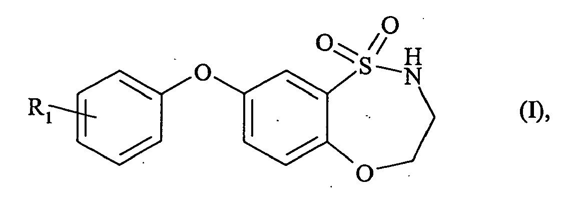 wherein R₁ represents a hydrogen atom, a cyano group, a linear or branched (C₁-C₆)alkoxycarbonyl group, a (C₁-C₆)alkylsulphonylamino-(Ci-C₆)alkyl group in which the alkyl moieties are each linear or branched, an N-hydroxycarboximidamide group or a heterocyclic group,
their enantiomers and diastereoisomers, and also addition salts thereof with a pharmaceutically acceptable acid or base,
it being understood that the term "heterocyclic group" denotes a 5-membered monocyclic aromatic group containing one to four hetero atoms which are the same or different and which are selected from nitrogen, oxygen and sulphur, it optionally being possible for said heterocyclic group to be substituted by one or more substituents which are the same or different and which are selected from linear or branched (C₁-C₆)alkyl and linear or branched (C₁-C₆)polyhaloalkyl.

2. Compounds of formula (I) according to claim 1, **characterised in that** R₁ represents a heterocyclic group.

3. Compounds of formula (I) according to claim 1, **characterised in that** R₁ represents a 5-membered monocyclic aromatic heterocyclic group containing at least one nitrogen atom, it optionally being possible for said group to be substituted by one or more substituents which are the same or different and which are selected from linear or branched (C₁-C₆)alkyl and linear or branched (C₁-C₆)polyhaloalkyl.

4. Compounds of formula (I) according to claim 1, **characterised in that** R₁ represents a pyrrolyl, pyrazolyl, imidazolyl, triazolyl, tetrazolyl, isoxazolyl, oxazolyl, isothiazolyl; thiazolyl, thiadiazolyl, dithiazolyl or oxadiazolyl group, it optionally being possible for each of the groups to be substituted by one or more substituents which are the same or different and which are selected from linear or branched (C₁-C₆)alkyl and linear or branched (C₁-C₆)polyhaloalkyl.

5. Compounds of formula (I) according to claim 1, **characterised in that** R₁ represents a thiazolyl or oxadiazolyl group, it optionally being possible for each of the groups to be substituted by one or more substituents which are the same or different and which are selected from linear or branched (C₁-C₆)alkyl and linear or branched (C₁-C₆)polyhaloalkyl.

6. Compounds of formula (I) according to claim 1, **characterised in that** R₁ represents a 1,3-thiazolyl or 1,2,4-oxadiazolyl group, it optionally being possible for each of the groups to be substituted by one or more substituents which are the same or different and which are selected from linear or branched (C₁-C₆)alkyl and linear or branched (C₁-C₆)polyhaloalkyl.

7. Compounds of formula (I) according to claim 1, **characterised in that** R₁ represents a 1,3-thiazolyl or 1,2,4-oxadiazolyl group substituted by a methyl or trifluoromethyl group.

8. Compounds of formula (I) according to claim 1, **characterised in that** R₁ is in meta position of the phenoxy ring carrying it.

9. Compounds of formula (I) according to claim 1, which are:
• 8-[3-(5-methyl-1,2,4-oxadiazol-3-yl)phenoxy]-3,4-dihydro-2H-5,1,2-benzoxathiazepine 1,1-dioxide;
• 8-[3-(3-methyl-1,2,4-oxadiazol-5-yl)phenoxy]-3,4-dihydro-2H-5,1,2-benzoxathiazepine 1,1-dioxide;
their enantiomers and diastereoisomers, and also addition salts thereof with a pharmaceutically acceptable acid or base.

10. Process for the preparation of compounds of formula (I) according to claim 1, **characterised in that** there is used as starting material a compound of formula (II): 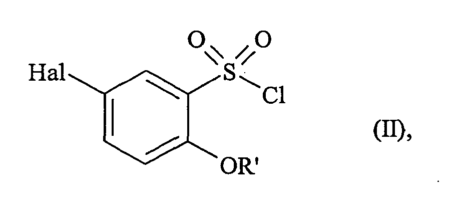 wherein Hal represents a halogen atom such as fluorine, chlorine or bromine and R' represents a linear or branched (C₁-C₆)alkyl group,
which is reacted with 2-chloroethylamine in a basic medium to yield the compound of formula (III): 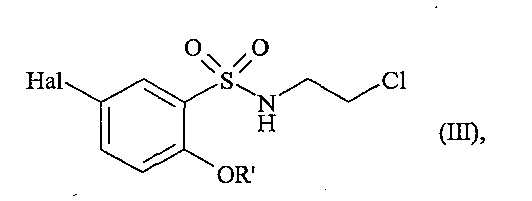 wherein Hal and R' are as defined hereinbefore,
which is then subjected to the action of a boron-containing compound to yield the compound of formula (IV): 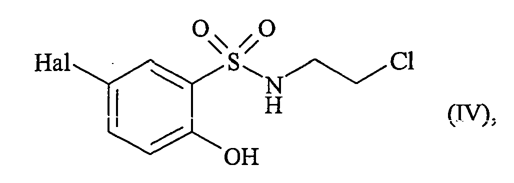 wherein Hal is as defined hereinbefore,
which is then cyclised to yield the compound of formula (V): 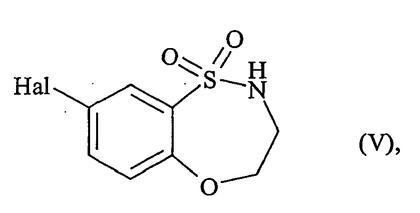 wherein Hal is as defined hereinbefore,
which is subjected to a reaction protecting the nitrogen atom to yield the compound of formula (VI): 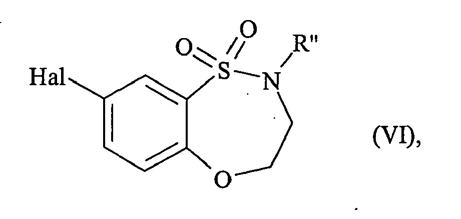 wherein Hal is as defined hereinbefore and R" represents a protecting group for the amine function such as, for example, a tert-butyloxycarbonyl group,
which is converted into a boronic acid to yield the compounds of formula (VII): 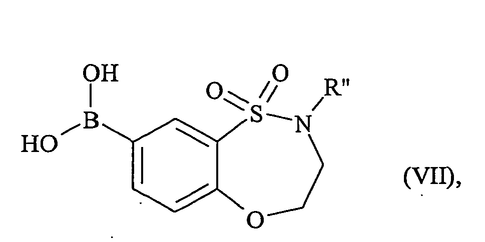 wherein R" is as defined hereinbefore,
which is reacted with an alcohol of formula (VIII): 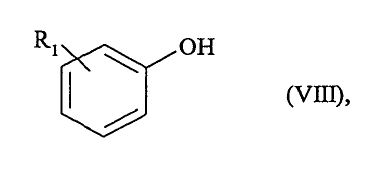 wherein R₁ is as defined for formula (I),
to yield the compound of formula (IX): 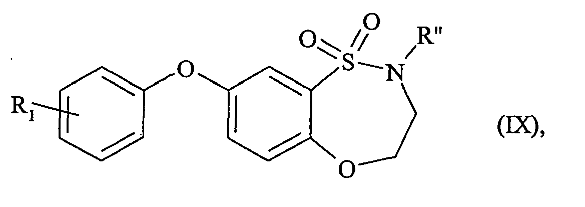 wherein R₁ and R" are as defined hereinbefore;
which is then subjected to a reaction deprotecting the amine function to yield the compound of formula (I),
a variant in the preparation of the compounds of formula (I) consisting of hydrolysis of the compound of formula (VII) to yield the compound of formula (X): 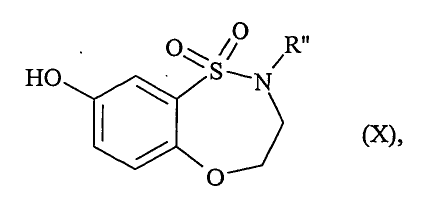 wherein R" is as defined hereinbefore,
which is reacted with a boronic acid compound of formula (XI): 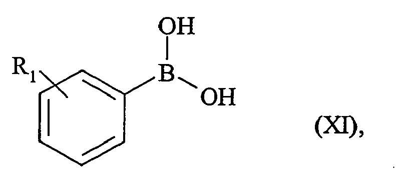 wherein R₁ is as defined for formula (I),
to yield the compound of formula (IX),
which is then subjected to a reaction deprotecting the amine function to yield the compound of formula (I),
another alternative in the preparation of the compounds of formula (I) consisting of using conventional chemical reactions, after preparation of the compounds of formula (IX), in order to subsequently change the substituent of the phenoxy ring,
which compound of formula (I) may then be purified according to a conventional separation technique, is converted, if desired, into its addition salts with a pharmaceutically acceptable acid or base and is separated, where appropriate, into its isomers according to a conventional separation technique.

11. Compounds of formula (V): 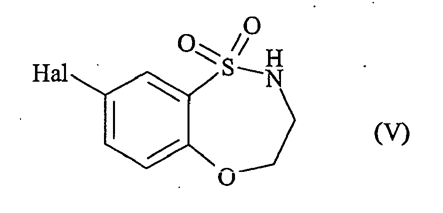 wherein Hal represents a halogen atom such as fluorine, chlorine or bromine,
**characterised in that** they are for use as synthesis intermediates for compounds of formula (I).

12. Pharmaceutical composition comprising as active ingredient a compound according to any one of claims 1 to 9 in combination with one or more inert, non-toxic, pharmaceutically acceptable carriers.

13. Pharmaceutical compositions according to claim 12 for use as modulators of the AMPA receptor.

14. Pharmaceutical compositions according to claim 12 for use in the treatment or prevention of disorders of memory and cognition that are associated with age, with syndromes of anxiety or depression, with progressive neurodegenerative diseases, with Alzheimer's disease, with Parkinson's disease, with Pick's disease, with Huntington's chorea, with Korsakoffs disease, with schizophrenia, with the sequelae of acute neurodegenerative diseases, with frontal lobe and subcortical dementias, with the sequelae of ischaemia and with the sequelae of epilepsy.

15. Use of compounds of formula (I) according to any one of claims 1 to 9 in the manufacture of medicaments for use as modulators of the AMPA receptor.

16. Use of compounds of formula (I) according to any one of claims 1 to 9 in the manufacture of medicaments for use in the treatment or prevention of disorders of memory and cognition that are associated with age, with syndromes of anxiety or depression, with progressive neurodegenerative diseases, with Alzheimer's disease, with Parkinson's disease, with Pick's disease, with Huntington's chorea, with Korsakoffs disease, with schizophrenia, with the sequelae of acute neurodegenerative diseases, with frontal lobe and subcortical dementias, with the sequelae of ischaemia and with the sequelae of epilepsy.

17. Compounds of formula (I) according to any one of claims 1 to 9 for use as modulators of the AMPA receptor.

18. Compounds of formula (I) according to any one of claims 1 to 9 for use in the treatment or prevention of disorders of memory and cognition that are associated with age, with syndromes of anxiety or depression, with progressive neurodegenerative diseases, with Alzheimer's disease, with Parkinson's disease, with Pick's disease, with Huntington's chorea, with Korsakoffs disease, with schizophrenia, with the sequelae of acute neurodegenerative diseases, with frontal lobe and subcortical dementias, with the sequelae of ischaemia and with the sequelae of epilepsy.

## Patentansprüche

1. Verbindungen der Formel (I): 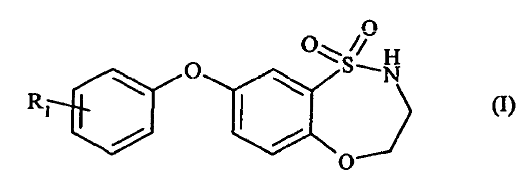 in der R₁ ein Wasserstoffatom, eine Cyanogruppe, eine geradkettige oder verzweigte (C₁-C₆-Alkoxycarbonylgruppe, eine geradkettige oder verzweigte (C₁-C₆)-Alkyl-(C₁-C₆)-sulfonylamonoalkylgruppe, eine N-Hydroxycarboximidamidgruppe oder eine heterocyclische Gruppe bedeutet,
deren Enantiomere und deren Diastereoisomere sowie deren Additionssalze mit einer pharmazeutisch annehmbaren Säure oder Base,
mit der Maßgabe, dass der Begriff heterocyclische Gruppe für eine aromatische monocyclische Gruppe mit 5 Kettengliedern steht, die ein bis vier identische oder verschiedene Heterotatome, ausgewählt aus Stickstoff, Sauerstoff und Schwefel enthält, wobei die heterocyclische Gruppe gegebenenfalls durch einen oder mehrere identische oder verschiedene Substituenten ausgewählt aus geradkettigem oder verzweigtem (C₁-C₆)-Alkyl oder geradkettigem oder verzweigtem (C₁-C₆)-Polyhalogenalkyl substituiert sein kann.

2. Verbindungen der Formel (I) nach Anspruch 1, **dadurch gekennzeichnet, dass** R₁ eine heterocyclische Gruppe bedeutet.

3. Verbindungen der Formel (I) nach Anspruch 1, **dadurch gekennzeichnet, dass** R₁ eine monocyclische, aromatische heterocyclische Gruppe mit 5 Kettengliedern bedeutet, die mindestens ein Stickstoffatom aufweist, wobei die Gruppe gegebenenfalls durch einen oder mehrere identische oder verschiedene Substituenten ausgewählt aus geradkettigem oder verzweigtem (C₁-C₆)-Alkyl oder geradkettigem oder verzweigtem (C₁-C₆)-Polyhalogenalkyl substituiert sein kann.

4. Verbindungen der Formel (I) nach Anspruch 1, **dadurch gekennzeichnet, dass** R₁ eine Pyrrolyl-, Pyrazolyl-, Imidazolyl-, Triazolyl-, Tetrazolyl-, Isoxazolyl-, Oxazolyl-, Isothiazolyl-, Thiazolyl-, Thiadiazolyl-, Dithiazolyl- oder Oxadiazolylgruppe bedeutet, wobei jede dieser Gruppen gegebenenfalls durch einen oder mehrere identische oder verschiedene Substituenten ausgewählt aus geradkettigem oder verzweigtem (C₁-C₆)-Alkyl oder geradkettigem oder verzweigtem (C₁-C₆)-Polyhalogenalkyl substituiert sein kann.

5. Verbindungen der Formel (I) nach Anspruch 1, **dadurch gekennzeichnet, dass** R₁ eine Thiazolyl- oder Oxadiazolylgruppe bedeutet, wobei jede dieser Gruppen gegebenenfalls durch einen oder mehrere identische oder verschiedene Substituenten ausgewählt aus geradkettigem oder verzweigtem (C₁-C₆)-Alkyl oder geradkettigem oder verzweigtem (C₁-C₆)-Polyhalogenalkyl substituiert sein kann.

6. Verbindungen der Formel (I) nach Anspruch 1, **dadurch gekennzeichnet, dass** R₁ eine 1,3-Thiazolyl- oder 1,2,4-Oxadiazolylgruppe bedeutet, wobei jede dieser Gruppen gegebenenfalls durch einen oder mehrere identische oder verschiedene Substituenten ausgewählt aus geradkettigem oder verzweigtem (C₁-C₆)-Alkyl oder geradkettigem oder verzweigtem (C₁-C₆)-Polyhalogenalkyl substituiert sein kann.

7. Verbindungen der Formel (I) nach Anspruch 1, **dadurch gekennzeichnet, dass** R₁ eine 1,3-Thiazolyl- oder 1,2,4-Oxadiazolylgruppe bedeutet, die durch eine Methyl- oder Trifluormethylgruppe substituiert ist.

8. Verbindungen der Formel (I) nach Anspruch 1, **dadurch gekennzeichnet, dass** R₁ in der meta-Stellung des sie tragenden Phenoxykerns gebunden ist.

9. Verbindungen der Formel (I) nach Anspruch 1, nämlich:
• 8-[3-(5-Methyl-1,2,4-oxadiazol-3-yl)-phenoxy]-3,4-dihydro-2H-5,1,2-benzoxathiazepin-1,1-dioxid;
• 8-[3-(3-Methyl-1,2,4-oxadiazol-5-yl)-phenoxy]-3,4-dihydro-2H-5,1,2-benzoxathiazepin-1,1-dioxid;
deren Enantiomere, deren Diastereoisolere sowie deren Additionssalze mit einer pharmazeutisch annehmbaren Säure oder Base.

10. Verfahren zur Herstellung der Verbindungen der Formel (I) nach Anspruch 1, **dadurch gekennzeichnet, dass** man als Ausgangsprodukt eine Verbindung der Formel (II) verwendet: 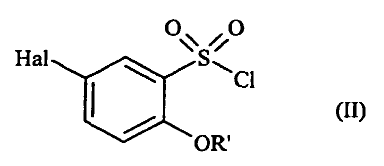 in der Hal ein Halogenatom, wie Fluor, Chlor oder Brom und R' eine geradkettige oder verzweigte (C₁-C₆)-Alkylgruppe bedeuten,
welche man mit 2-Chlorethylamin in basischem Medium umsetzt zur Bildung der Verbindung der Formel (III): 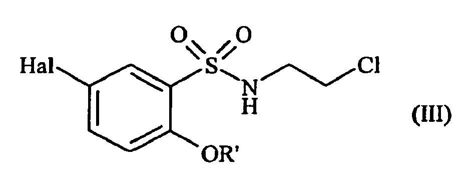 in der Hal und R' die oben angegebenen Bedeutungen besitzen,
welche man der Einwirkung eines Borderivats unterwirft zur Bildung der Verbindung der Formel (IV): 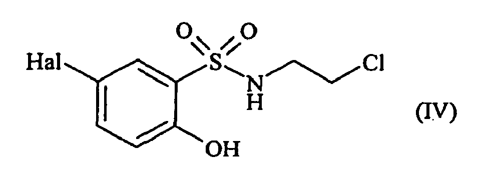 in der Hal die oben angegebenen Bedeutungen besitzt,
welche anschließend cyclisiert wird zur Bildung der Verbindung der Formel (V): 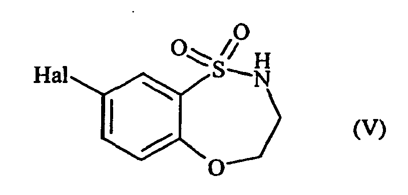 in der Hal die oben angegebenen Bedeutungen besitzt,
welche man einer Reaktion zum Schutz des Stickstoffatoms unterzieht zur Bildung der Verbindung der Formel (VI): 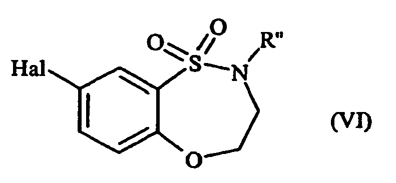 in der Hal die oben angegebenen Bedeutungen besitzt und R" eine Schutzgruppe für die Aminfunktion darstellt, wie beispielsweise die tert.-Butyloxycarbonylgruppe,
welche man in die Boronsäure umwandelt zur Bildung der Verbindungen der Formel (VII): 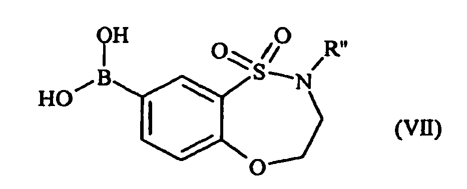 in der R" die oben angegebenen Bedeutungen besitzt,
welche man mit einem Alkohol der Formel (VIII) umsetzt: 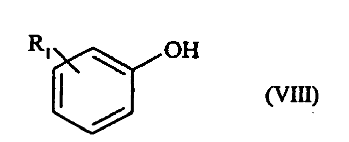 in der R₁ die bezüglich der Formel (I) angegebenen Bedeutungen besitzt,
zur Bildung der Verbindung der Formel (IX): 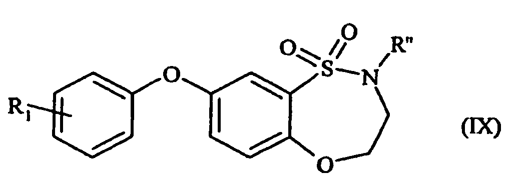 in der R₁ und R" die oben angegebenen Bedeutungen besitzen,
welche anschließend einer Reaktion zur Abspaltung der Schutzgruppe der Aminfunktion unterworfen wird zur Bildung der Verbindung der Formel (I),
wobei eine Variante der Herstellung der Verbindungen der Formel (I) in der Hydrolyse der Verbindung der Formel (VII) besteht zur Bildung der Verbindung der Formel (X): 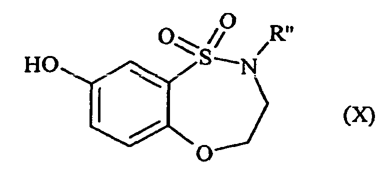 in der R' die oben angegebenen Bedeutungen besitzt,
welche man mit einem Boronsäurederivat der Formel (XI) umsetzt: 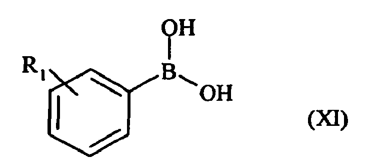 in der R₁ die bezüglich der Formel (I) angegebenen Bedeutungen besitzt,
zur Bildung der Verbindung der Formel (IX);
welche anschließend einer Reaktion zur Abspaltung der Schutzgruppe der Aminfunktion unterworfen wird zur Bildung der Verbindung der Formel (I),
wobei eine weitere Alternative der Herstellung der Verbindungen der Formel (I) darin besteht, nach der Herstellung der Verbindungen der Formel (IX) in einer zweiten Stufe unter Anwendung von klassischen Reaktionen der Chemie den Substituenten des Phenoxykerns zu modifizieren,
welche Verbindung der Formel (I) mit Hilf einer klassischen Trennungsmethode gereinigt werden kann, welche man gewünschtenfalls in ihre Additionssalze mit einer pharmazeutisch annehmbaren Säure oder Base überführt und deren Isomeren man gegebenenfalls mit Hilfe einer klassischen Trennungsmethode trennt.

11. Verbindungen der Formel (V): 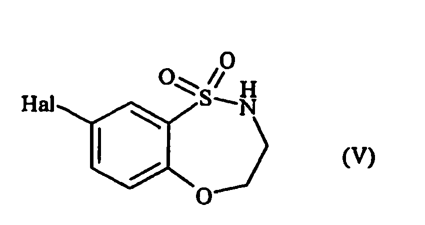 in der Hal ein Halogenatom, wie Fluor, Chlor oder Brom bedeutet,
**dadurch gekennzeichnet, dass** sie nützlich sind als Zwischenprodukte bei der Synthese der Verbindungen der Formel (I).

12. Pharmazeutische Zubereitung enthalten als Wirkstoff eine Verbindung nach einem der Ansprüche 1 bis 9 in Kombination mit einem oder mehreren inerten, nichttoxischen und pharmazeutisch annehmbaren Hilfsstoffen.

13. Pharmazeutische Zubereitungen nach Anspruch 12, nützlich als Modulatoren des AMPA-Rezeptors.

14. Pharmazeutische Zubereitungen nach Anspruch 12, nützlich bei der Behandlung oder der Vorbeugung von mnemokognitiven Störungen, die mit dem Alter verknüpft sind, Angst- oder Depressionssyndromen, progressiven neurodegenerativen Erkrankungen, der Alzheimerschen Krankheit, der Parkinsonschen Krankheit, der Pickschen Krankheit, der Huntingtonchorea, der Korsakoffkrankheit, der Schizophrenie, den Folgen von akuten neurodegenerativen Erkrankungen, von frontalen und subkortikalen Demenzen, von Folgen der Ischämie und von Folgen der Epilepsie.

15. Verwendung der Verbindungen der Formel (I) nach einem der Ansprüche 1 bis 9 für die Herstellung von Arzneimitteln, die nützlich sind als Modulatoren des AMPA-Rezeptors.

16. Verwendung der Verbindungen der Formel (I) nach einem der Ansprüche 1 bis 9 für die Herstellung von Arzneimitteln, die nützlich sind zur Behandlung oder Vorbeugung von mnemokognitiven Störungen, die mit dem Alter verknüpft sind, Angst- oder Depressionssyndromen, progressiven neurodegenerativen Erkrankungen, der Alzheimerschen Krankheit, der Parkinsonschen Krankheit, der Pickschen Krankheit, der Huntingtonchorea, der Korsakofflcrankheit, der Schizophrenie, den Folgen von akuten neurodegenerativen Erkrankungen, von frontalen und subkortikalen Demenzen, von Folgen der Ischämie und von Folgen der Epilepsie.

17. Verbindungen der Formel (I) nach einem der Ansprüche 1 bis 9, nützlich als Modulatoren des AMPA-Rezeptors.

18. Verbindungen der Formel (I) nach einem der Ansprüche 1 bis 9, nützlich zur Behandlung oder Vorbeugung von mnemokognitiven Störungen, die mit dem Alter verknüpft sind, Angst- oder Depressionssyndromen, progressiven neurodegenerativen Erkrankungen, der Alzheimerschen Krankheit, der Parkinsonschen Krankheit, der Pickschen Krankheit, der Huntingtonchorea, der Korsakoffkrankheit, der Schizophrenie, den Folgen von akuten neurodegenerativen Erkrankungen, von frontalen und subkortikalen Demenzen, von Folgen der Ischämie und von Folgen der Epilepsie.
